# EUROPÄISCHE PATENTSCHRIFT

(11) **EP 0 638 315 B1**
(45) Veröffentlichungstag und Bekanntmachung des Hinweises auf die Patenterteilung: **18.11.1998**
(21) Anmeldenummer: 93113006.6
(22) Anmeldetag: 13.08.1993
(51) Int. Cl.: A61K 38/41

(54) **Antihypoxisches Mittel**
Antihypoxic agent
Agent antihypoxique

(43) Veröffentlichungstag der Anmeldung: 15.02.1995
(73) Patentinhaber: Karsanov, Nikolai Vasilievich, Tbilisi (GE)
(72) Erfinder: Karsanov, Nikolai Vasilievich, Tbilisi (GE); Kipshidze, Nodar Nikolaievich, Tbilisi (GE); Selikhova, Evgenia Vasilievna, Tbilisi (GE); Guchua, Eteri Ivlianovna, Digomsky massiv (GE)
(74) Vertreter: Patentanwälte Zellentin & Partner

(56) Entgegenhaltungen:
- DATABASE WPI Week 8904, Derwent Publications Ltd., London, GB; AN 89-029135 & JP-A-63 303 927 (TAKEO S) 12. Dezember 1988
- DATABASE WPI Week 8208, Derwent Publications Ltd., London, GB; AN 82-14607E & JP-A-57 007 408 (FREUNT SANGYO KK ET AL) 14. Januar 1982
- CHEMICAL ABSTRACTS, vol. 86, no. 7, 14. Februar 1977, Columbus, Ohio, US; abstract no. 37558q, A. A. SURMENKOV 'Effect of cytochrome c on the course of local myocardial hypoxia'
- CHEMICAL ABSTRACTS, vol. 112, no. 5, 29. Januar 1990, Columbus, Ohio, US; abstract no. 30416p, V. I. KOSTIN 'Preclinical estimation of the antianginal activity of energy metabolism intermediates'
- CHEMICAL ABSTRACTS, vol. 80, no. 13, 1. April 1974, Columbus, Ohio, US; abstract no. 68936s, R. NUNEZ ET AL 'Nicotinamide coenzymes in heart and coronary blood during myocardial infarction'

## Beschreibung

Die vorliegende Erfindung betrifft die Medizin und bezieht sich insbesondere auf die Schaffung von Mitteln, welche den energetischen Metabolismus wiederherstellen und damit hypoxische Zustände von Geweben, Herz, Gehirn, Leber u.a. aufheben.

Als Prototyp ist ein bekannter Antihypoxant, nämlich Zytochrom C gewählt.

Durch einmalige Verabreichung von Zytochrom C in einer Dosis von 2,5 mg/kg Tierkörpergewicht an Hunden mit einer reproduzierten sechsstündigen Koronararterienokklusion wird bei ihnen 15 Minuten nach dieser Okklusion die Gewebeatmung verbessert und im Ischämiegebiet die Entwicklung nekrotischer Erscheinungen in bedeutendem Masse verhindert, aber kein Einfluss auf die Grösse des ischämischen Gebiets ausgeübt (A. Zalevsky et. al. Am. Heart J., 1987, v. 113 no 1 pp. 124-129).

Die Dosissteigerung auf 5 mg/kg und besonders auf 10 mg/kg bei einer 2 Stunden lang dauernden Koronararterienokklusion führt zu einer Erhöhung des Gehaltes an Adenosintriphosphorsäure (ATP) und Creatinphosphorsäure (CrP) in der extraischämischen Gegend des Myokards auf den Normalwert und zu einer wesentlichen Zunahme des Gehalts der genannten Bestandteile, aber nicht auf den Normalwert. Dadurch ergibt sich wiederum eine verbesserte Struktur und Funktion des Myokards (N.V. Karsanov u.a. Materialy Respublikanskoi-konferentzii po enzimologii /Materialien der wissenschaftlichen Republikkonferenz über die Enzymologie/, Tbilissi, Juni 1981).

Wenn man bei Koronararterienokklusion Zytochrom C 1 bis 7 Tage bei täglicher Dosis von 10 mg/kg anwendet, erfolgt jedoch nach der Erhöhungsphase des Gehalts an Adenylnukleotiden auf den Normalwert (1-3 Tage) wieder eine Senkung des Gehalts derselben auf die Kontrollgrösse am siebenten Tag (Sh.B. Igrashev, N.M. Juldashev: Pharmakol. Toxikologie, 1988, Nr. 5, S. 41 bis 44).

Durch Anwendung von Zytochrom C in einer Dosis von 10 mg/kg bei einer Adrenalin-Koffein-Myokarditis, an der Kaninchen leiden, wird bei diesen der Gehalt an ATP und CrP während 7 Tage normalisiert (N.V. Karsanov, N.K. Khaindrava: Materialy II Vsesojuznogo simpoziuma po meditsinskoi enzimologii /Materialien des II. Allunionssymposiums der medizinischen Enzymologie/, Duschanbe, 1974).

Jedoch wird der Gehalt an ADP (Adenosindiphosphorsäure) und AMP (Adenosinmonophosphorsäure) im Vergleich zur Kontrolle nicht erhöht. Dies bewirkt eine Vergrösserung des Verhältnisses von ATP zu ADP und führt als Folge davon zu einer endogenen Hemmung der Aktivität des Krebszyklus, besonders dann, wenn der Gehalt an ATP den Normalwert übersteigt.

Wendet man Zytochrom C in einer beträchtlich höheren Dosis von 20 mg/kg bei einem bei Menschen auftretenden grossherdigen Myokardinfarkt an, welcher durch keine Insuffizienz der Kontraktionstätigkeit des Herzens und durch keine klinisch ausgeprägte Störung des Koronarkreislaufs kompliziert ist, dann wird beim Menschen der Zustand des Myokards der um den Infarkt herumliegenden Zone verbessert und die Grösse des nekrotischen Bezirks vermindert (B.N. Bezborodko, I.A. Shekhunova: Vrach. delo, 1990, Nr. 8, S. 8 bis 10).

Somit wird sowohl bei einmaliger als auch bei einer länger dauernden Verabreichung von Zytochrom C eine wesentliche Verbesserung des energetischen Zustandes des Ischämiegebiets des Myokards bewirkt, aber zu keiner ausgeglichenen stabilen Beseitigung des Energiedefizits der Zelle beigetragen und keine vollständige Wiederherstellung der Homeostasis des Energieversorgungssystems gewährleistet.

Aus Derwent AN-89-029135 (JP-A-63303927) sind Injektionslösungen bekannt, welche Adenosin, Inosin und Hypoxanthin enthalten und zur Infarktprophylaxe und zur Behandlung ischämischer Anfälle dienen.

Aus Derwent AN-82-14607 E (JP-A-57007408) ist eine feste Zytochrom C-Zubereitung bekannt, welche dünndarmlöslich ist, und zur Behandlung von Dyspnoe (Atemstörungen) und Sauerstoffmangel dient.

Aus CA 86(7): 37558q (Surmenkov, A.A., Kislorodn, Rezhim Tkanei (1974) S. 124-31) sind Injektionslösungen von Zytochrom C zur Behandlung lokaler myokardialer Hypoxie bekannt.

CA 112(5): 30416p (Kostin, V.I., Farmakol. Toksikol. (Moscow) (1989), 52(6), S. 49-52) beschreibt eine Zubereitung aus NAD und Malat mit antianginöser Wirkung nach Verschluß der Koronararterien.

Aus CA 89(13): 68936s (Nunez, R. et al, Amer. J. Physiol. (1974), 226(1), S. 73-76) sind Verfahren zur Bestimmung von Nicotinamidcoenzymen (NAD, NADH, NADP und NADPH) bei Myokardinfekten bekannt.

Die vorliegende Erfindung bezweckt die Entwicklung eines Mittels, das eine Kombination von Bestandteilen aufweist. welche auf die Aufhebung eines (hypoxischen) Energiemangel-Zustandes in der Zelle, auf die Wiederherstellung der Homeostasis des Energieversorgungssystems durch harmonische Beeinflussungen desselben und auf die Verkürzung von Zeiten für des Erreichen einer stabilen pharmakologischen Wirkung. z.B. im Falle einer Myokarditis und einer Koronararterienokklusion gerichtet ist.

Die gestellte Aufgabe wird dadurch gelöst, dass in die Zusammensetzung des pharmakologischen Mittels zwecks Stimulierung von bei hypoxischen Zuständen am stärksten schädigbaren drei Gliedern eines komplizierten Systems zur Energieversorgung der Zelle, welche Glieder jeweils für die Glykolyse (es wird die glykolytische Oxydoreduktion vermindert), für die oxydative Phosphorilierung (es wird die Leistung des Transports von Elektronen über die Übertragungskette zum Sauerstoff hin herabgesetzt) und für die Synthese von Adenylnukleotiden de novo verantwortlich sind, Reizmittel, d.h. Zytochrom C (Ferment), Nikotinamid-Adenin-Dinukleotid (NAD) als Koferment und Inosin (ein Metabolit, der ein Produkt beim Abbau von Adenylnukleotiden darstellt) eingeführt sind wobei die genannten Komponenten in einem folgenden Massenverhältnis (Gew. Teile)

| | |
|---|---|
| Zytochrom C | 5 bis 15, |
| Nikotin-Adenin-Dinukleotid | 0,5 bis 5, |
| Inosin | 20 bis 250. |

genommen werden.

Eine bevorzugte Zusammensetzung ist die folgende:

| | |
|---|---|
| Zytochrom | 10 |
| NAD | 0,5 |
| Inosin | 80 |

Das erfindungsgemäß bevorzugte antihypoxische Mittel der obengenannten Zusammensetzung, welches im weiteren als "ENERGOSTIM" bezeichnet wird, wirkt nicht durch die Summation von Effekten, sondern als pharmakologisches Mittel, das eine neue Eigenschaft, d.h. eine Fähigkeit besitzt, die Funktion sämtlicher Glieder im Energieversorgungssystem, z.B. die Homeostasis des Systems, die in einer Pathologie gestört und bei einseitiger Einwirkung kompliziert wird, wiederherzustellen.

Die Störung des Homeostasis des Energieversorgungssystems bei hypoxischen Zuständen der Zelle und die dadurch verursachte Entwicklung eines Energiemangel-Zustandes der Zelle treten aus dem Grunde auf, dass die Zelle beträchtliche Mengen an Zytochrom C und NAD (s. Karsanov u.a., 1981 und Tabelle 1) verliert sowie der gesamte Gehalt an Adenylnukleotiden (Tabelle 2) vermindert wird.

Durch Verminderung der Menge an Zytochrom C in Mitochondrien ergibt sich eine herabgesetzte Intensität des Transportes (eines Stroms) von Elektronen zum Sauerstoff hin und wird damit die Intensität der Synthese von ATP in der mit dem Transport von Elektronen gekoppelten Reaktion der oxidativen Phosphorylierung vermindert.

Die Verminderung der Synthese von ATP in der Herzmuskelzelle geschieht auch im Zusammenhang mit der Abnahme des Gehaltes an NAD in der Zelle (sowohl im Zytosol als auch in den Mitochondrien). Dies führt zu einer Verminderung der Intensität der Bildung von ATP während der Glykolyse sowie der oxydativen Phosphorylierung einerseits durch Verringerung des Transportes von Protonen aus dem Zytosol zu den Mitochondrien hin (über den Malat-Asparat-Shunt) infolge der Herabsetzung der Aktivität glykolytischer NAD-abhängigen Dehydrogenasen, der glykolytischen Oxydoreduktase und andererseits durch die Herabsetzung der Aktivität der NAD-abhängigen Dehydrogenasen des Krebszyklus.

Die Verabreichung von Zytochrom C bei hypoxischen Zuständen der Zelle führt zum Einbau von Zytochrom C in die zum Transport von Elektronen zum Sauerstoff hin dienende Übertragungskette in den Mitochondrialmembranen (Karsanov-u.a., 1981) und damit zur Wiederherstellung von Elektronenströmen über deren Übertragungskreis. Dadurch wird die Synthese von ATP durch die oxydative Phosphorilierung aktiviert. Damit wird der Gehalt an Adenosintriphosphorsäure (ATP), die eine Quelle einer für die Verwertung leicht zugänglichen Energie ist, erhöht und deren Vorrat als CrP auf das normale Niveau oder auf ein fast normales Niveau (Tabelle 3) gesteigert. Jedoch wird dabei der Gehalt and ADP und AMP nicht wesentlich (bei allergischer Myokarditis) erhöht, während der gesamte Gehalt an Adenylnukleotiden niedrig (bei toxikoallergischer Myokarditis, Tabelle 2) bleibt oder etwas (bei Adrenalinschädigung des Herzens, Tabelle 4) gesteigert oder sogar normalisiert (bei allergischer Myokarditis, Tabelle 3).

Jedoch übt die Verabreichung von Zytochrom C auf das Verhältnis von [NAD] zu [NADH] keinen Einfluss aus (d.h. es bleibt zu NADH hin verschoben), obwohl der gesamte Gehalt an Pyridinnukleotiden eigentlich steigt (Tabelle 1). Im Zusammenhang damit kann man annehmen, dass unter Einfluss von Zytochrom C die Aktivität der glykolytischen NAD-abhängigen Dehydrogenasan und der NAD-abhängigen Dehydrogenasen des Krebszyklus wesentlich nicht geändert wird.

Also findet unter Einwirkung von Zytochrom C allein keine vollständige Wiederherstellung der Homeostasis des Systems zur Energieversorgung der Zelle statt.

Durch Anwendung des einzigen NAD allein wird der Gehalt an ATP im Myokard auf das normale Niveau (bei allergischer Myokarditis und bei Adrenalinschädigung des Herzens, Tabellen 3 und 4) erhöht oder auf ein Niveau (bei toxikoallergischer Myokarditis, Tabelle 2) gesteigert, das nahe dem normalen Niveau liegt.

Da die Verabreichung von NAD zu einer vollständigen Normalisierung des Gehaltes und Verhältnisses von NAD/NADH (Tabellen 4, 5) führt, gibt es Gründe, anzunehmen, dass die Normalisierung des Gehaltes an ATP durch die Aktivierung der glykolytischen NAD-abhängigen Dehydrogenasen, der glykolytischen Oxydoreduktion und des Transportes von Proton aus dem Zytosol in die Mitochondrien sowie durch die Aktivierung der NAD-abhängigen Dehydrogenasen des Krebszyklus geschieht. Jedoch ist als Hauptweg zur Aktivierung der Synthese von ATP in diesem Fall der glykolytische Weg anzusehen, weil dabei der Gehalt an CrP im Unterschied von der Anwendung von Zytochrom C bei Adrenalinschädigung des Herzens (Tabelle 4) in keinem beachtlichen Masse zunimmt. Das ist doch verständlich, weil die Durchsatzleistung der zum Transport von Elektronen dienenden Übertragungskette (aus Mangel an Zytochrom C) gering bleibt.

Was ADP betrifft, so hat der ADP-Gehalt eine Tendenz zu steigen. Dadurch sinkt das [ATP]/[ADP]-Verhältnis bei Anwendung von NAD allein (s. Tabellen 2, 3, 4).

Somit wird durch Anwendung von NAD allein wie auch durch Anwendung von Zytochrom allein keine sichere Wiederherstellung der Homeostasis des Systems zur Energieversorgung der Zelle (trotz der Zunahme von ATP auf den Normalwert oder auf einen diesem naheliegenden Wert) erzielt. In beiden Fällen findet auch keine vollständige Wiederherstellung der Ultrastruktur der Herzmuskelzelle (Tabelle 5) statt. Durch Anwendung von Inosin allein bei allergischer Myokarditis (Tabelle 6) im Unterschied von der Anwendung von Zytochrom C und NAD werden nicht der Gehalt an ATP, sondern im wesentlichen die Gehalte an AMP und ADP normalisiert. Der Gehalt der letzteren beiden Komponenten, besonders der ADP-Gehalt übersteigt bei einer höheren Inosindosis (160 mg/kg Körpergewicht) sogar das normale Niveau. Bei Hunden mit einer Koronararterienokklusion (Tabelle 7) geschieht eine wesentliche Erhöhung des Gehaltes an ADP auch bei einer optimalen Inosindosis (80 mg/kg Tierkörpergewicht) und ebenfalls bei keiner Zunahme von ATP, was nicht nur im Ischämiegebiet sondern auch in der extraischämischen Gegend der linken Herzkammer der Fall ist. Auf diese Weise wird das ATP/ADP-Verhältnis bei Anwendung von Inosin allein gegenüber dem Normalwert noch stärker (Tabellen 6, 7) verringert. Die Zunahme von ADP und AMP ist so wesentlich, dass der gesamte Gehalt an Adenylnukleotiden auf den Normalwert und sogar oberhalb desselben steigt. Dies lässt sich dadurch erklären, dass Inosin einen ausgeprägten Einfluss auf die Synthese von Adenylnukleotiden de novo ausübt.

Das oben Gesagte zeugt davon, dass durch Inosin allein wie auch durch Zytochrom allein oder durch NAD allein das Energieversorgungssystem bei entzündlichen Schädigungen des Myokards und einer Koronararterienokklusion nicht in den Zustand der Homeostasis zurückgebracht wird. Die Anwendung des kombinierten erfindungsgemässen Mittels, das NAD, Zytochrom C und Inosin in einem optimalen Verhältnis zueinander umfasst und als "ENERGOSTIM" bezeichnet ist, trägt bei einer toxikoallergischen Mykarditis und einer Koronararterienokklusion zu einer vollständigen Normalisierung aller Indizes des Energieversorgungssystems der Zelle (Tabellen 8, 9) bei. Dabei ergibt sich keine Summation von Effekten, sondern eine qualitativ neue Wirkung, d.h. eine vollständige Wiederherstellung der Homeostasis des komplizierten Systems zur Energieversorgung der Zelle, was nicht erreichbar ist, wenn man die obengenannten Präparate im einzelnen anwendet. Würde unter Einwirkung von ENERGOSTIM eine blosse Summation von Effekten stattfinden, so würde der Gehalt an ATP nicht normalisiert, sondern würde er das normale Niveau um das zweifache und mehr darüber übersteigen. Dasselbe würde für die anderen Indizes des Zustands der Energieversorgung gelten.

Bei Anwendung von ENERGOSTIM wird nicht beobachtet, dass der Gehalt an ADP oberhalb des Normalwertes steigt, was bei Anwendung von Inosin allein festgestellt wird.

Wenn man berücksichtigt, dass die Gehalte an ATP, ADP und CrP sowie das Verhältnis dieser Gehaltswerte, d.h. [ATP]/[ADP], [ADP] [CrP] [P_{anorgan}]/[ATP] [CrP] und NAD/NADH, die Intensität der einen oder der anderen Vorgänge regeln (aktivieren, hemmen), welche im komplizierten Energieversorgungssystem ablaufen, wird die Wichtigkeit der Wiederherstellung der Homeostasis des Systems offensichtlich. Vielmehr stabilisiert der normale Gehalt an ATP und ADP verschiedene Strukturen der Zelle und bestimmt den Zustand kontraktiler Eiweisse.

Durch einen Mangel an ATP wird z.B. das Myokard in einen Kontrakturzustand gebracht, während ein hoher Gehalt an ATP in Abwesenheit von Ca das Myokard in einen atonischen Zustand bringt.

Man vermischte Zytochrom C (10 mg/kg), NAD (0,5 mg/kg) und Inosin (80 mg/kg Tierkörpergewicht) miteinander und löste das so erhaltene Gemisch bei sorgfältigem Rühren in 5 ml einer physiologischen vorgewärmten (38-40°C), fabrikmässig hergestellten Lösung unmittelbar vor Anwendung.

### Beispiel 1.

### Pharmakologische Wirkung des erfindungsgemässen ENERGOSTIM bei toxikoallergischer Myokarditis (TAM) (Verhältnis der Bestandteile: 0,5 mg/kg NAD, 10 mg/kg Zytochrom C, 80 mg/kg Inosin).

Man führte Versuche an Kaninchen durch. Man reproduzierte TAM und AM (allergische Myokarditis) nach den Methoden von S.V. Andreev und M.V. Sokolov (s. im Buch "Modelirovanie zabolevanii (Simulation von Erkrankungen)", Moskau, 1973). Zur Reproduzierung von TAM wurden intravenös in einem Abstand von 4 Tagen 2 mal je 2 ml Pferdeblutserum und 7 Tage nach der letzten Injektion des genannten Serums 0,5 ml Staphylokkkentoxin injiziert.

Die pharmakologische Wirkung des erfindungsgemässen ENERGOSTIM bei 10-tägiger TAM registrierte man nach 5-tägiger Kur der intravenösen Verabreichung des erfindungsgemässen Präparates in einer täglichen Dosis von 90,5 mg/kg Tiergewicht, die ab dem fünften Tag der Erkrankungsentwicklung begann. Um die Wirkung des erfindungsgemässen ENERGOSTIM mit der pharmakologischen Wirkung von Zytochrom C vergleichen zu können, wurde Zytochrom C ebenfalls intravenös in einer Dosis 10 mg/kg Tiergewicht bei Behandlungskuren entsprechender Dauer (Tabelle 11) verabreicht.

Der Grad und Charakter des Einflusses der zu untersuchenden Mittel auf den hypoxischen Energiemangel-Zustand wurde nach dem Gehalt an ATP, ADP, AMP, CrP, dem ATP/ADP-Verhältnis und nach der energetischen Ladung des Systems von Adenylnukleotiden beurteilt.

Bei einer TAM von 10-tägiger Dauer ergibt ENERGOSTIM bei 5-tägiger Verabreichungskur eine ausgeprägte pharmakologische Wirkung, d.h. normalisiert den Gehalt an Adenylnukleotiden und deren Summe, die energetische Ladung des Systems und den Gehalt an CrP in völligem Masse (s. Tabelle 8). Zur selben Zeit führt bei AM (allergischer Myokarditis) die Verabreichung von Zytochrom allein in einer Dosis von 10 mg/kg während 7 Tage zu keiner vollständigen Wiederherstellung der Leistung des Energieversorgungssystems, des homeostatischen Zustandes desselben.

Die Wiederherstellung der Synthese von Makroergen in dem komplizierten Energieversorgungskreis, welche mit einer Verbesserung des Blutkreislaufs im Myokard vereint wird, führt zu einer wesentlichen Verbesserung der Ultrastruktur des Myokards, zu einer schnellen Rückwärtsentwicklung und Organisation eines Entzündungsprozesses, zu einer Reparation der Struktur des Myokards.

Dies äussert sich in der Verringerung des intrazellulären Ödems, im Füllen der intermyofibrillösen Räume mit Mitochondrien und in einer Erhöhung der Dichte der Matrix der Mitochondrien. Dabei erwerben die Mitochondrien eine ovale Form, ihre Kristen (Cristae) werden dicker und deren Anzahl erreicht die Norm; es wird die Zweikreisstruktur der Mitochondrialmembranen wiederhergestellt. Es wird der Gehalt an Glykogenkörnchen erhöht; es wird die Geordnetheit in der Anordnung von Z-Leisten wiederhergestellt, die Anzahl von übermässig kontrahierten Sarkomeren wesentlich vermindert; es wird der Erweiterungsgrad der Kanälchen des sarkoplasmatischen Reticulums verringert.

Die wesentliche Verbesserung der Struktur des Myokards bei Anwendung von ENERGOSTIM führt nach den Befunden der intrakardialen Hämodynamik zur Wiederherstellung der systolischen und diastolischen Funktionen des Herzens, d.h. dazu, dass der systolische Druck praktisch auf den Normaldruck steigt und seine normale Anstiegsgeschwindigkeit erreicht wird und der diastolische Druck auf die Norm sinkt.

Daraus kann man schlussfolgern, dass durch das erfindungsgemässe ENERGOSTIM bei Energiemangel-Zuständen, TAM die Energieversorgung (energetische Tätigkeit) des Herzmuskels vollständig harmonisch wiederhergestellt, die Entwicklung eines Entzündungsprozesses unterdrückt und damit die funktionelle Aktivität des Myokards erhöht und die Zeiten für das Erreichen einer stabilen pharmakologischen Wirkung ohne Entwicklungsgefahr von Intoxikationserscheinungen verkürzt werden.

### Beispiel 2.

### Pharmakologische Wirkung des erfindungsgemässen ENERGOSTIM bei einer akuten fokalen Ischämie bei Hunden (Verhältnis der Bestandteile: 0,5 mg/kg NAD, 10 mg/kg Zytochrom, 80 mg/kg Inosin).

Es wurde eine fokale Myokardischämie bei mischrassigen Hunden durch Abbindung des absteigenden Astes der Koronararterie in dessen oberem Drittel unter Äthernarkose mit der Prämedikation mit einem Gemisch von Fentanyl-Dimedrol-Droperidol (Initialnarkose mit Natriumthiopental) und eine künstliche Ventilation der Lungen mit atmosphärischer Luft mit Ätherdämpfen über einen Respirator PO-5 reproduziert. Es wurde ENERGOSTIM in einer Dosis von 90,5 mg/kg Tiergewicht und Zytochrom C in einer Dosis von 10 mg/kg verabreicht. Man verwendete ein zu untersuchendes Material, das 2 Stunden nach der Reproduzierung der Koronararterienokklusion entstand. Die Anwendung von ENERGOSTIM gestattet es, den Gehalt an Adenylnukleotiden und deren Verhältnis vollständig wiederherzustellen, den Gehalt an CrP ausserhalb des Ischämiegebiets zu normalisieren und im Ischämiegebiet um 29% gegenüber der Kontrolle (Tabelle 9) zu erhöhen. Unter Einwirkung von ENERGOSTIM übersteigt die durch die im Ischämiegebiet liegenden glyzerinisierten Fasern des Myokards entwickelte Spannung die Kontrollgrösse um das 1,74-fache, erreicht aber nicht den normalen Pegel und wird hingegen die in dem ausserhalb des Ischämiegebiets liegenden Bereich auftretende Spannung auf fast die Norm erhöht. Dabei wird die Ultrastruktur der Herzmuskelzelle wesentlich verbessert sowie werden der maximale systolische Druck, die Drucksenkungsgeschwindigkeit in der linken Herzkammer und der Index der Erschlaffung des Myokard (Tabelle 10) normalisiert. Durch Verabreichung von Zytochrom C allein wird wie auch bei Myokarditiden die Struktur der Mitochondrien verbessert und der Gehalt an ATP und CrP erhöht, jedoch zu keiner vollständigen Aufhebung von Energiemangel-Zuständen und zu keiner vollständigen Wiederherstellung der systolischen und diastolischen Funktionen des Herzens beigetragen, wenn man auch die Verabreichung von Zytochrom C vor der Reproduzierung der Koronararterienokklusion begann.

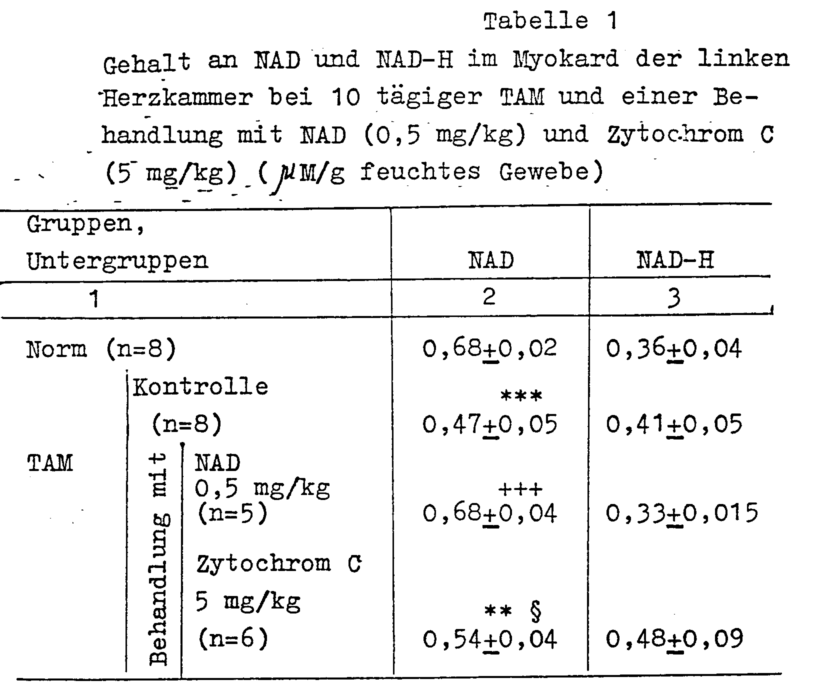

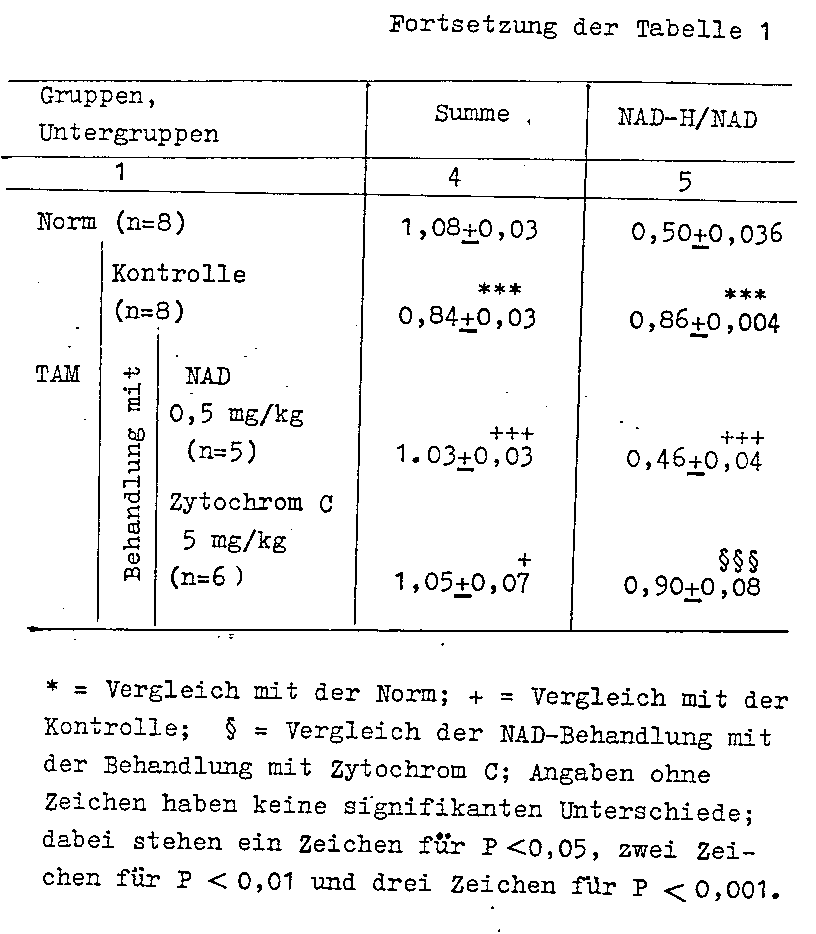

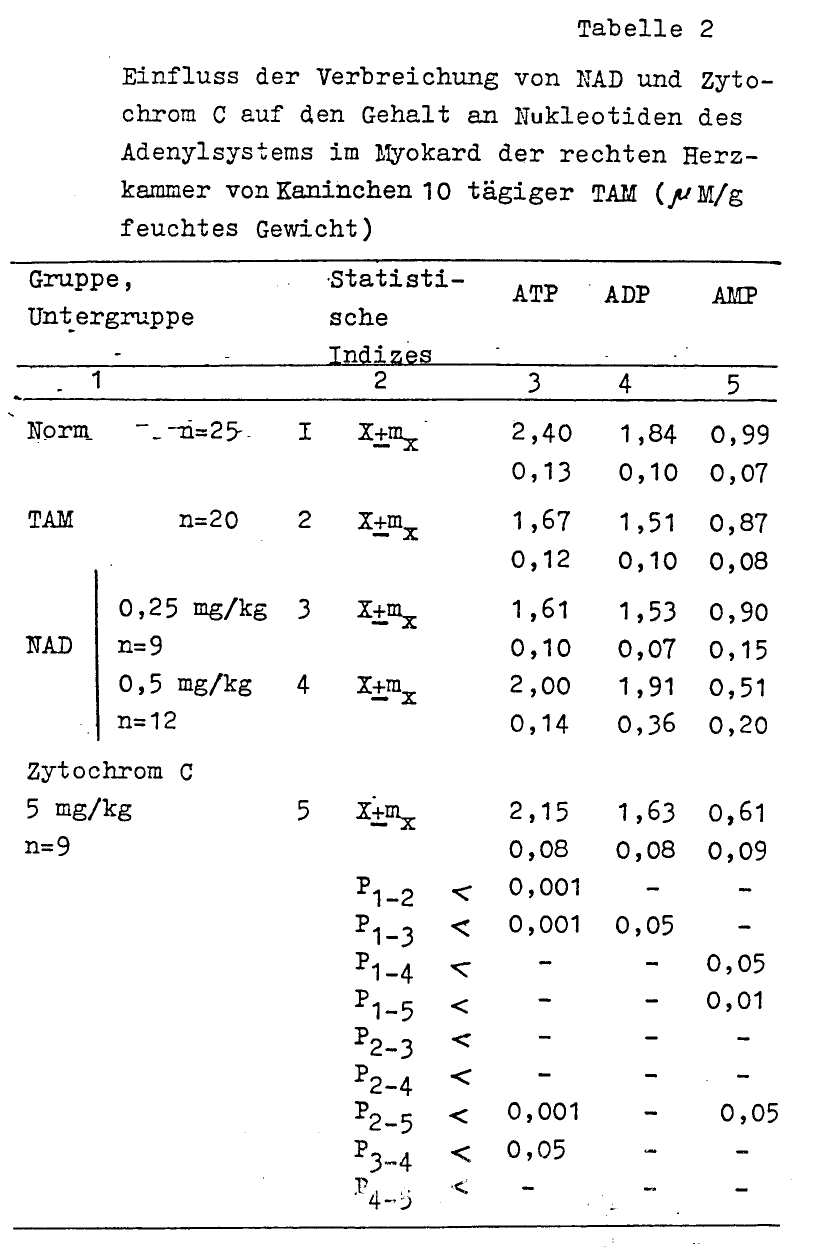

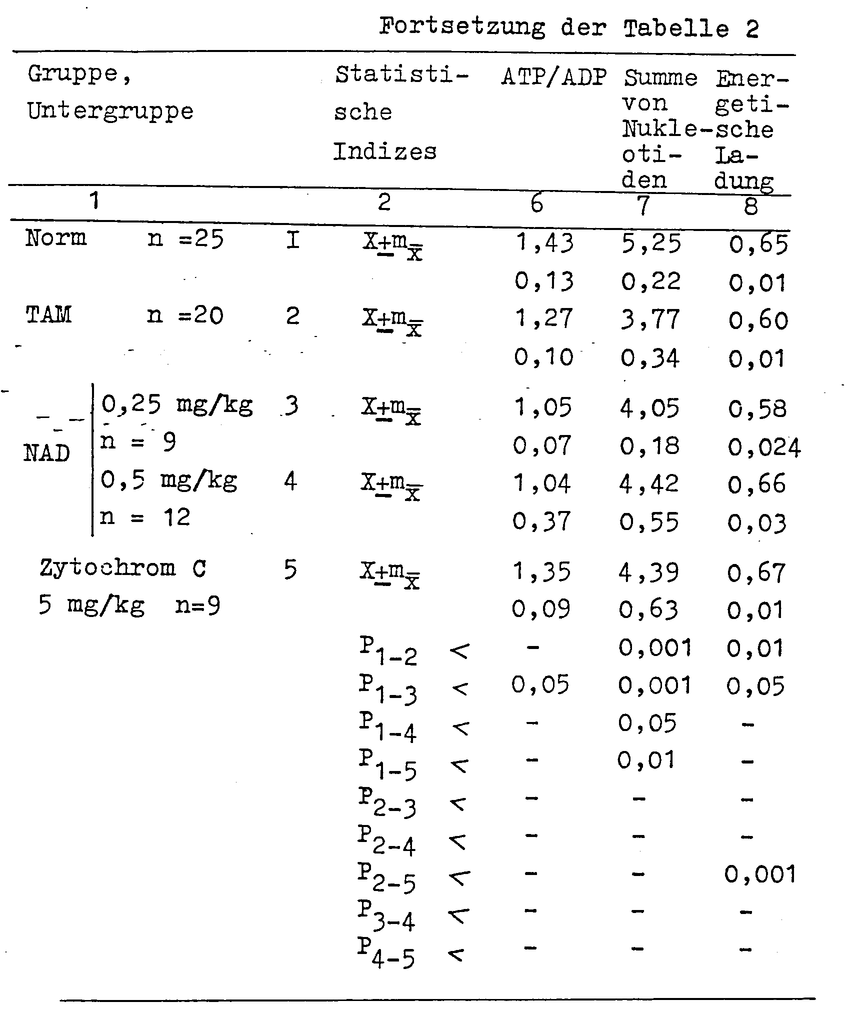

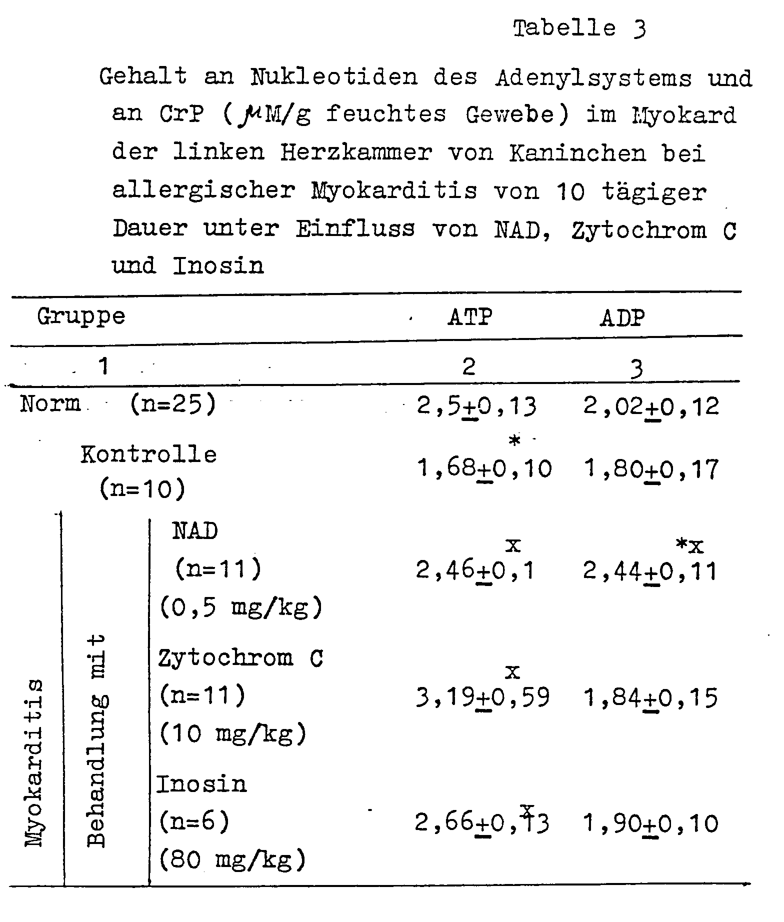

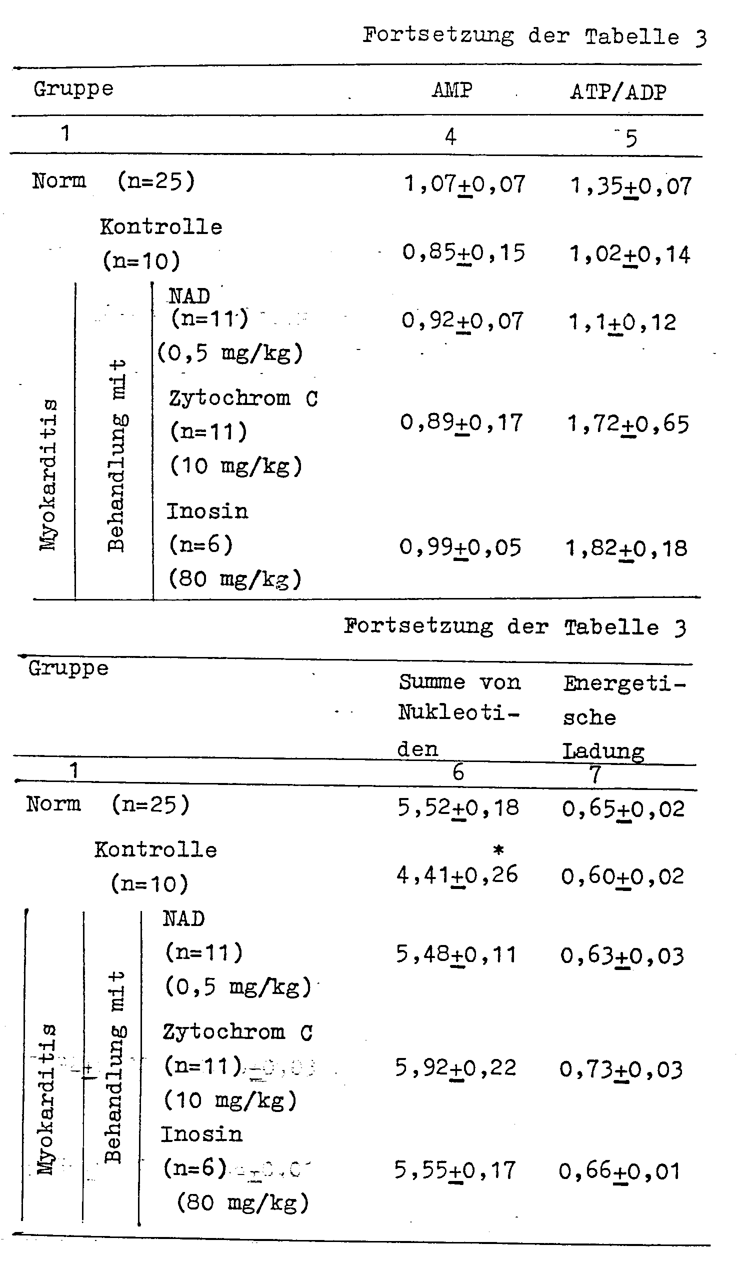

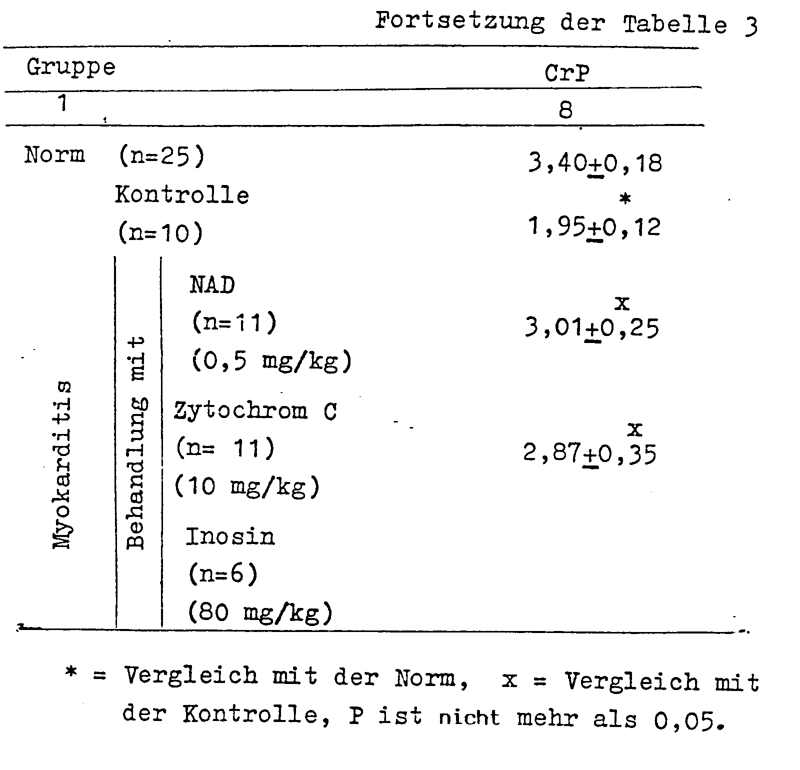

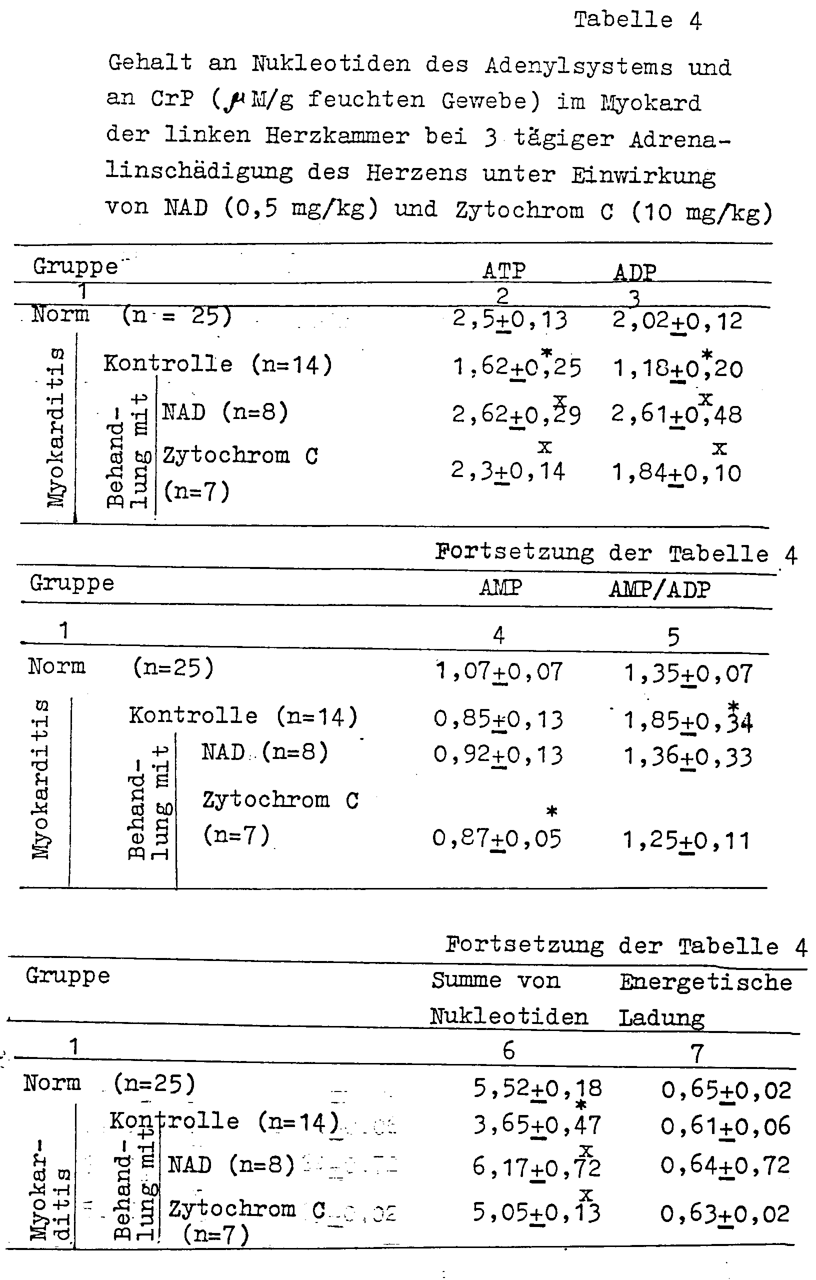

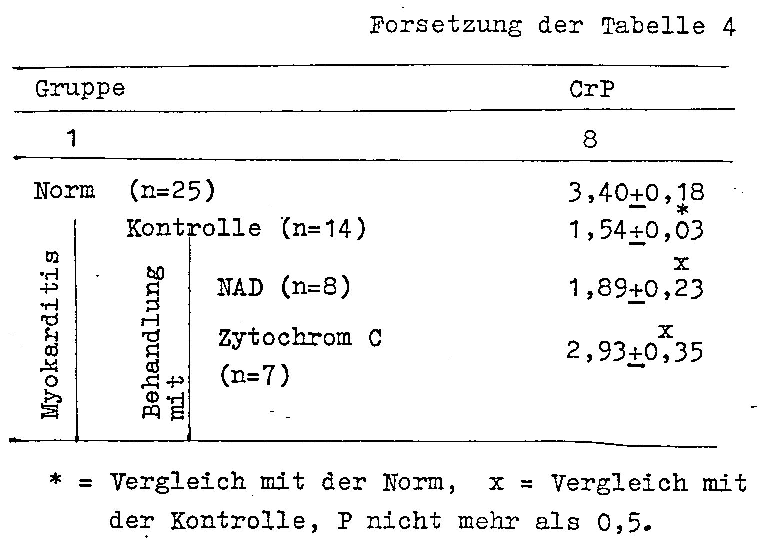

**Tabelle 5**

| Quantitative Analyse von Mitochondrien aufgrund von Elektronenbeugungsdiagrammen (EBD) der linken Herzkammer von normalen Kaninchen, Kaninchen mit 10- tägiger TAM und denen mit einer TAM, die mit NAD (0,5 mg/kg) und Zytochrom C (5 mg/kg) behandelt wurden | | |
|---|---|---|
| Kenndaten | Intakte Kaninchen (n=8) | TAM |
| | | Kontrolle (n=3) |
| 1 | 2 | 3 |
| Mitochondrien-Zahl in einem EBD | 20,0±0,72 | 7,0±0,48*** |
| Anzahl von Cristae in einem EBD | 193,5±24,7 | 27,0±4,0*** |
| Flächeninhalt von Mitochondrien in einem EBD (µm²) | 3,96±0,25 | 5,46±0,5*** |
| Flächeninhalt eines Mitochondriums (µm²) | 0,15±0,019 | 0,66±0,13*** |
| Anzahl von Cristae in einem Mitochondrium | 10,0±0,27 | 4,0±0,57*** |
| Energetischer Wirkungskoeffizient der Mitochondrien | 766±55 | 191±38*** |

| Kenndaten | TAM | |
|---|---|---|
| | NAD (0,5 mg/kg) (n=3) | Zytochrom C (6 mg/kg) (n=3) |
| 1 | 4 | 5 |
| Mitochondrien-Zahl in einem EBD | 10,35±2,12** | 12,26±1,11**++ |
| Anzahl von Cristae in einem EBD | 65,4±5,6**++ | 56,4±1,81***++ |
| Flächeninhalt von Mitochondrien in einem EBD (µm²⁾ | 5,0±0,20* | 4,64±0,19* |
| Flächeninhalt eines Mitochondriums (µm²) | 0,5±0,062** | 0,38±0,014***+ |
| Anzahl von Cristae in einem Mitochondrium | 5,2±0,9** | 4,6±2,64* |
| Energetischer Wirkungskoeffizient der Mitochondrien | 354±54,7**+ | 334,4±23,47**++ |

| | | |
|---|---|---|
| * = Vergleich mit der Norm; | | |
| + = Vergleich mit der Kontrolle, wobei ein Zeichen für P <0,05, zwei Zeichen für P <0,01 und drei Zeichen für P < 0,001 stehen. | | |

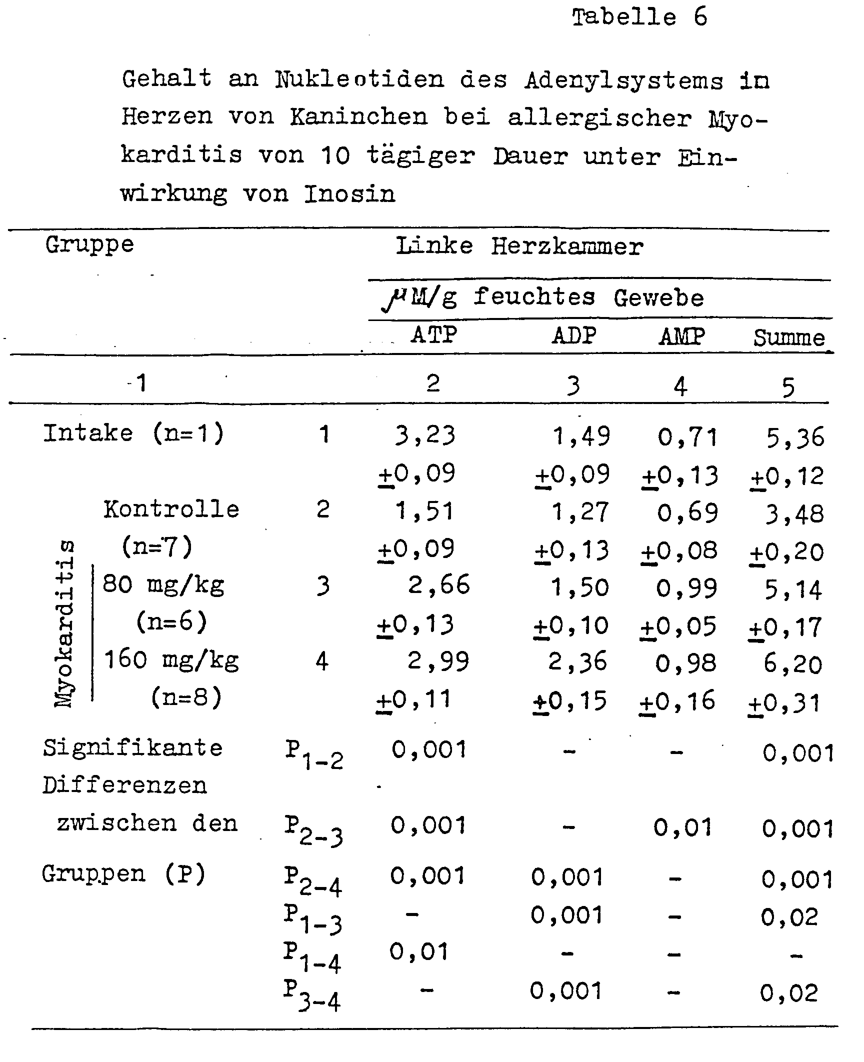

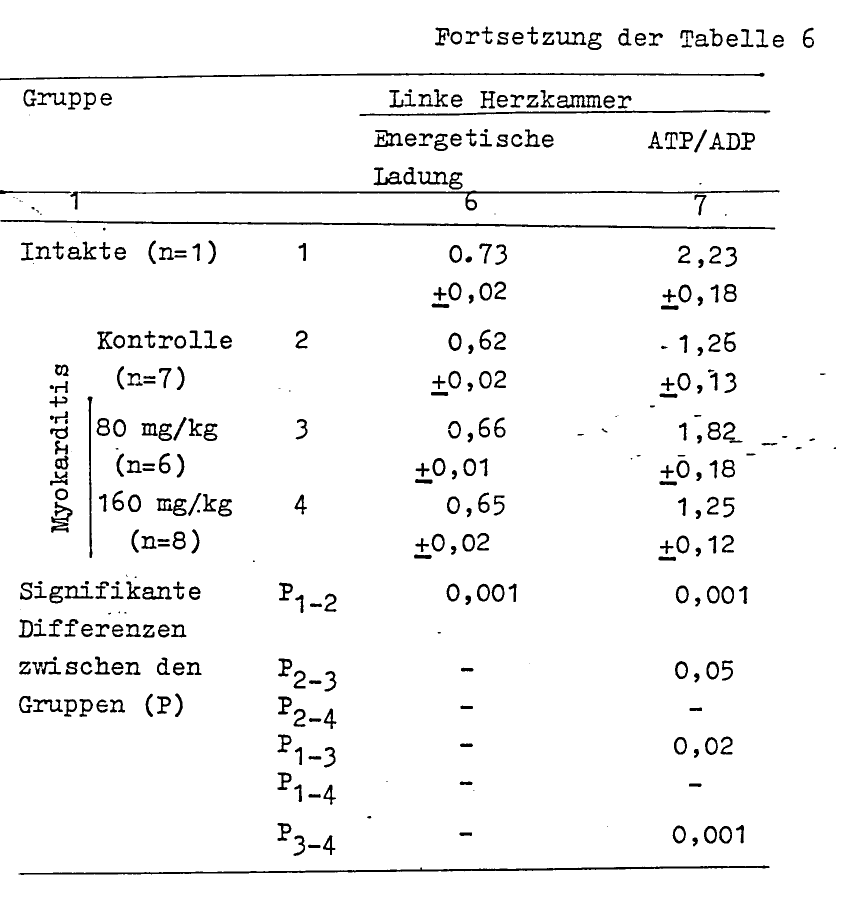

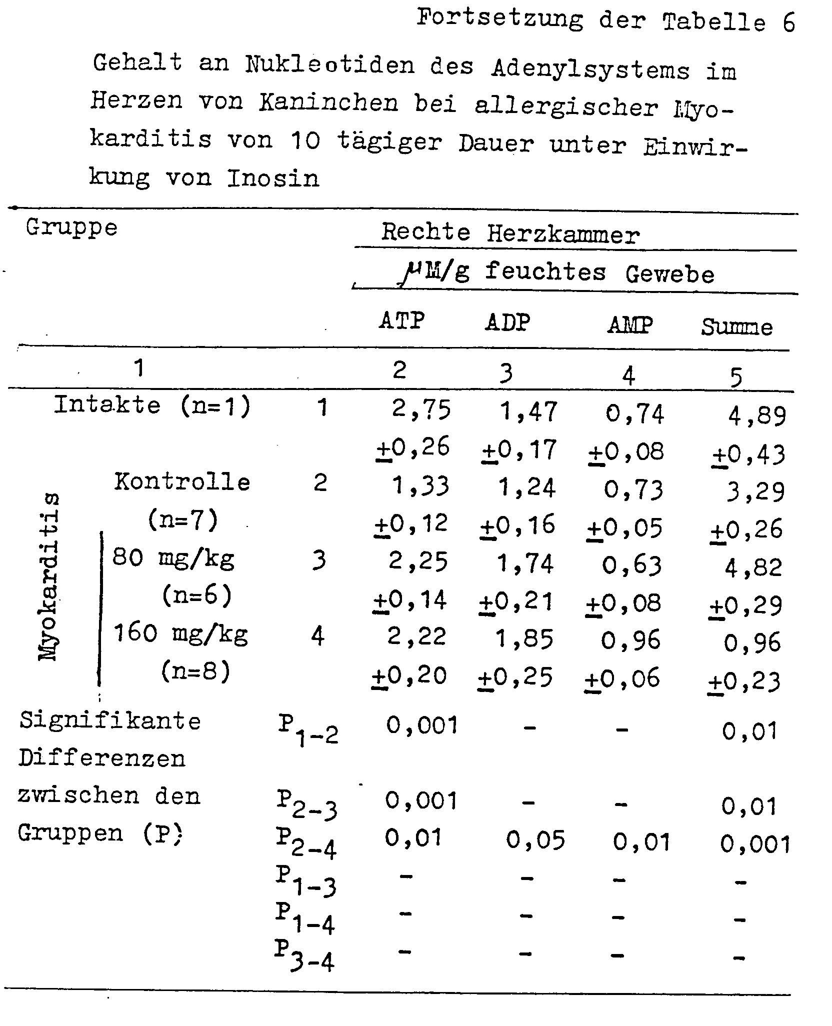

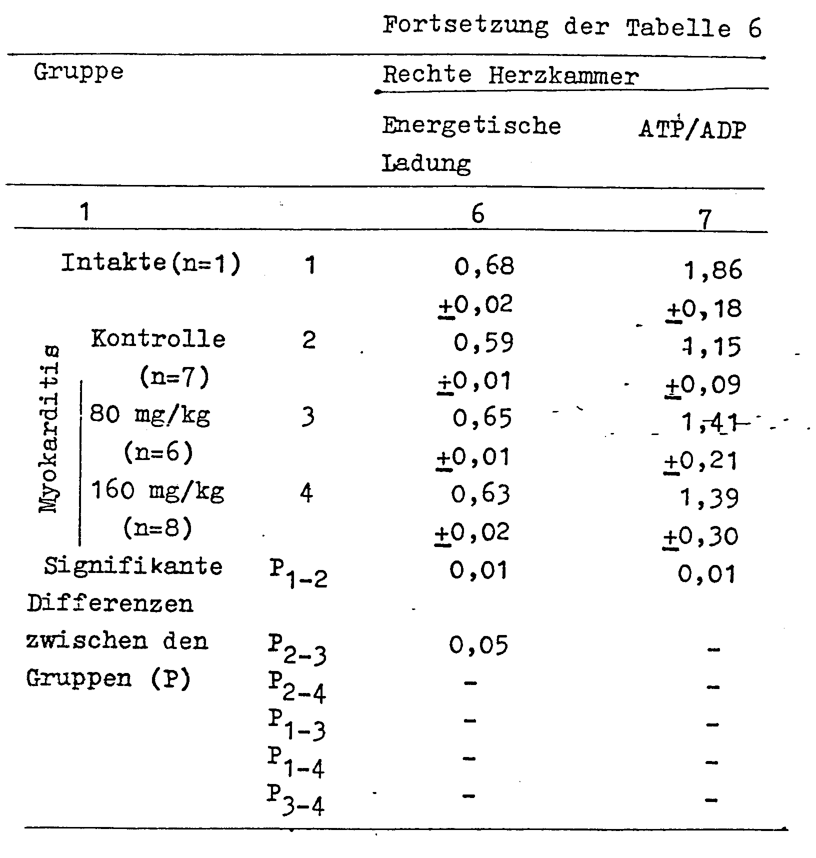

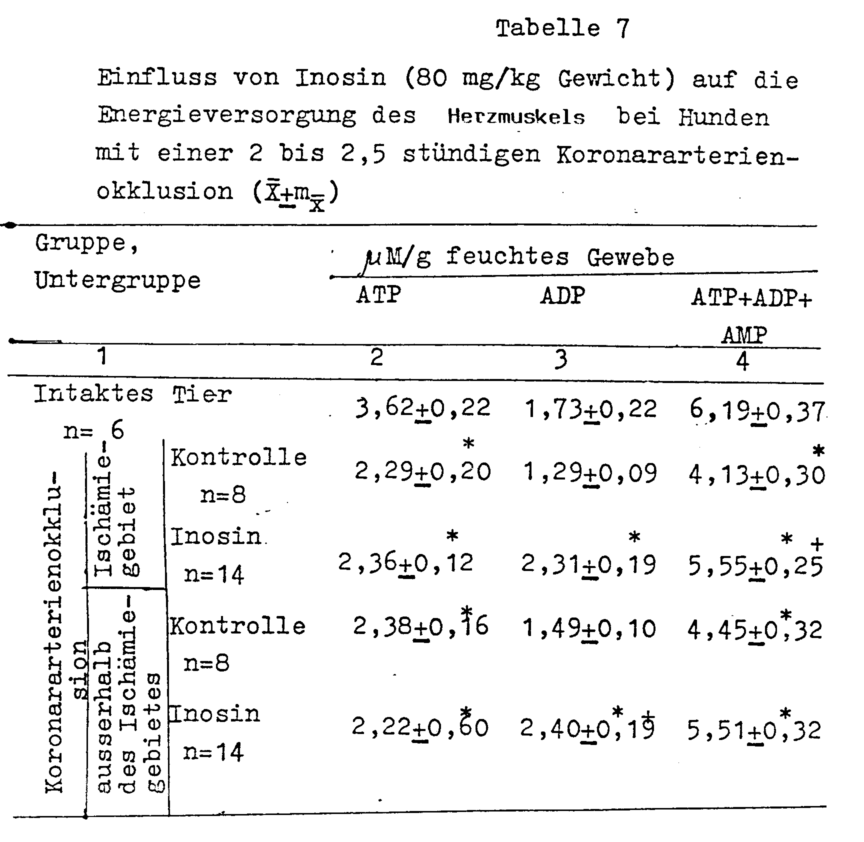

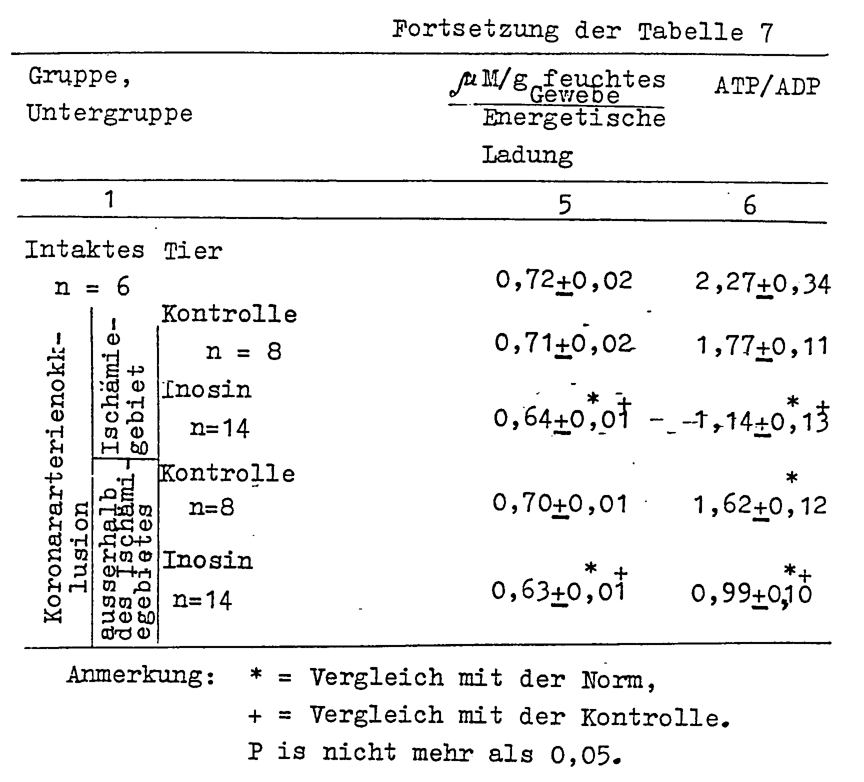

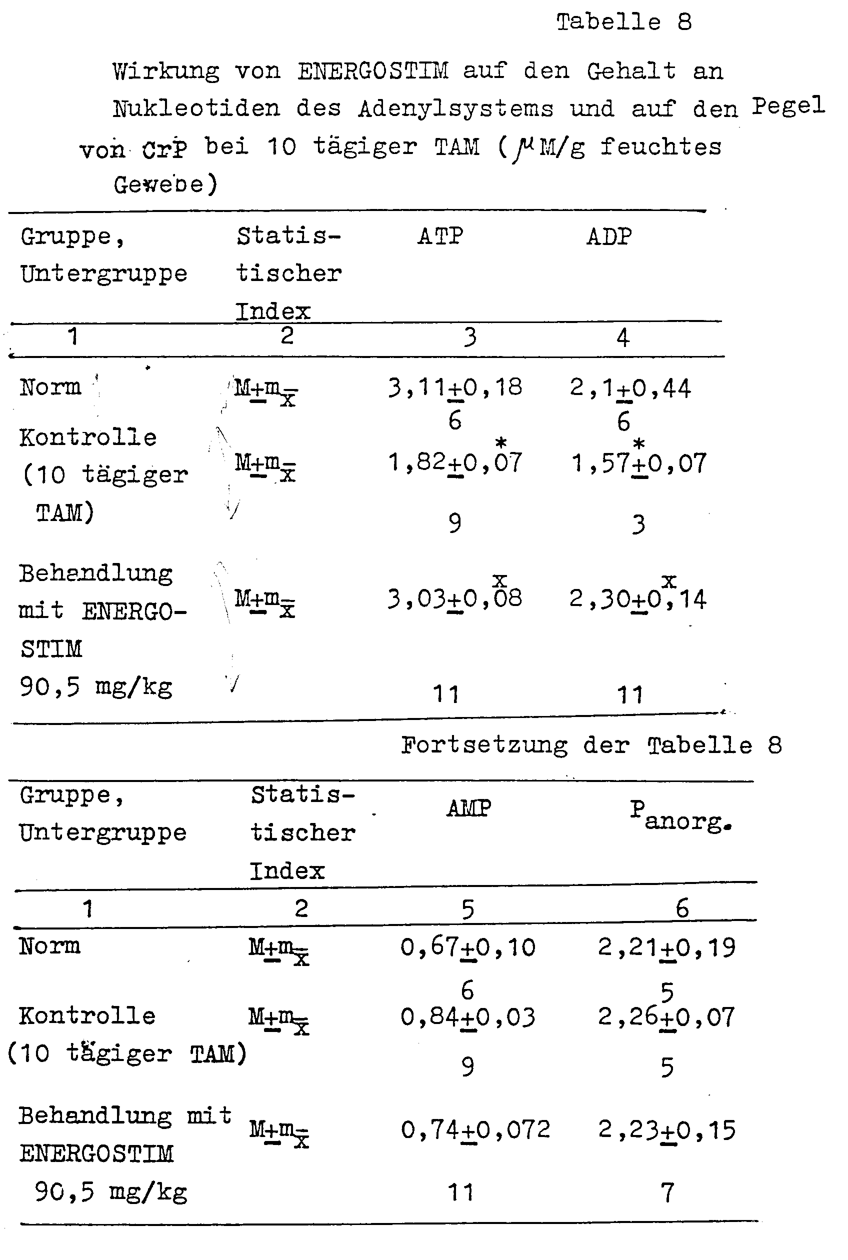

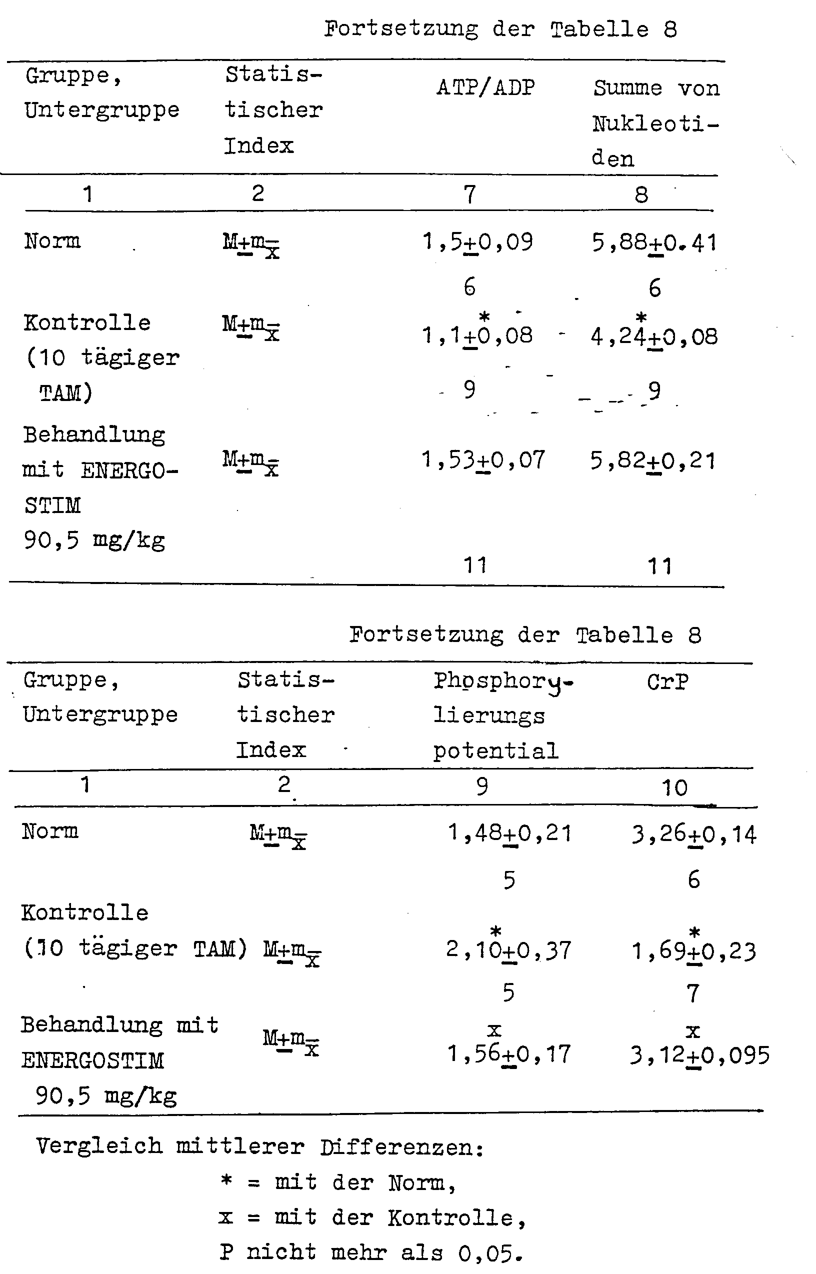

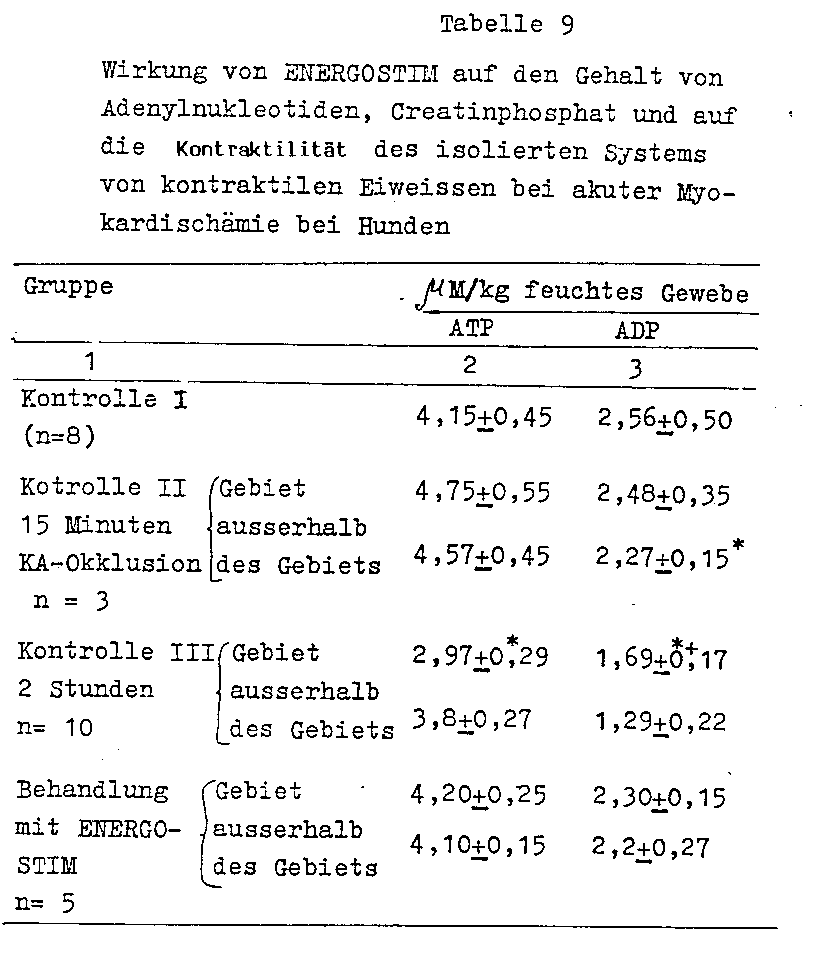

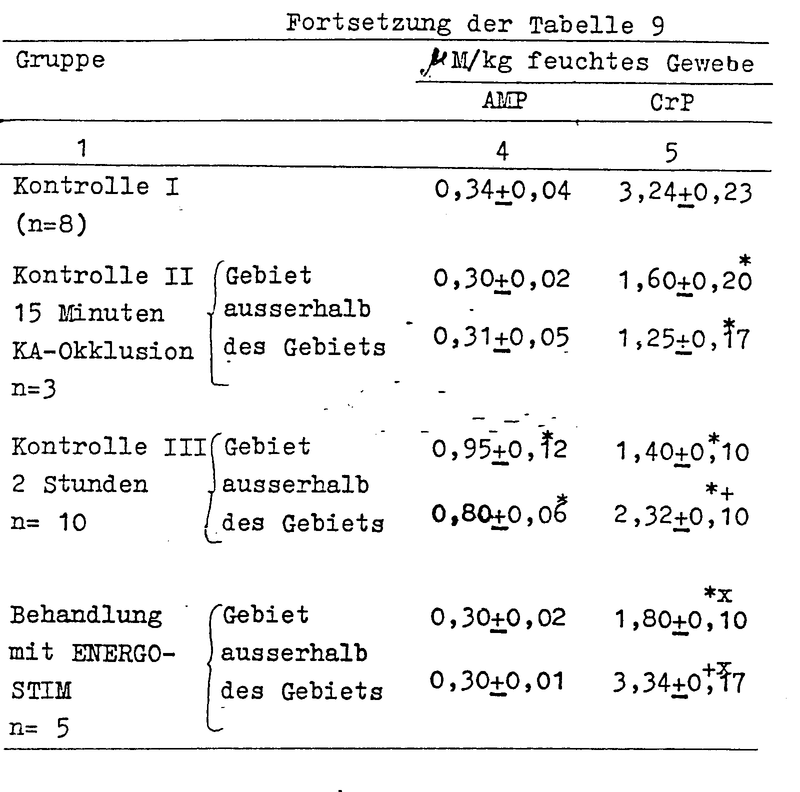

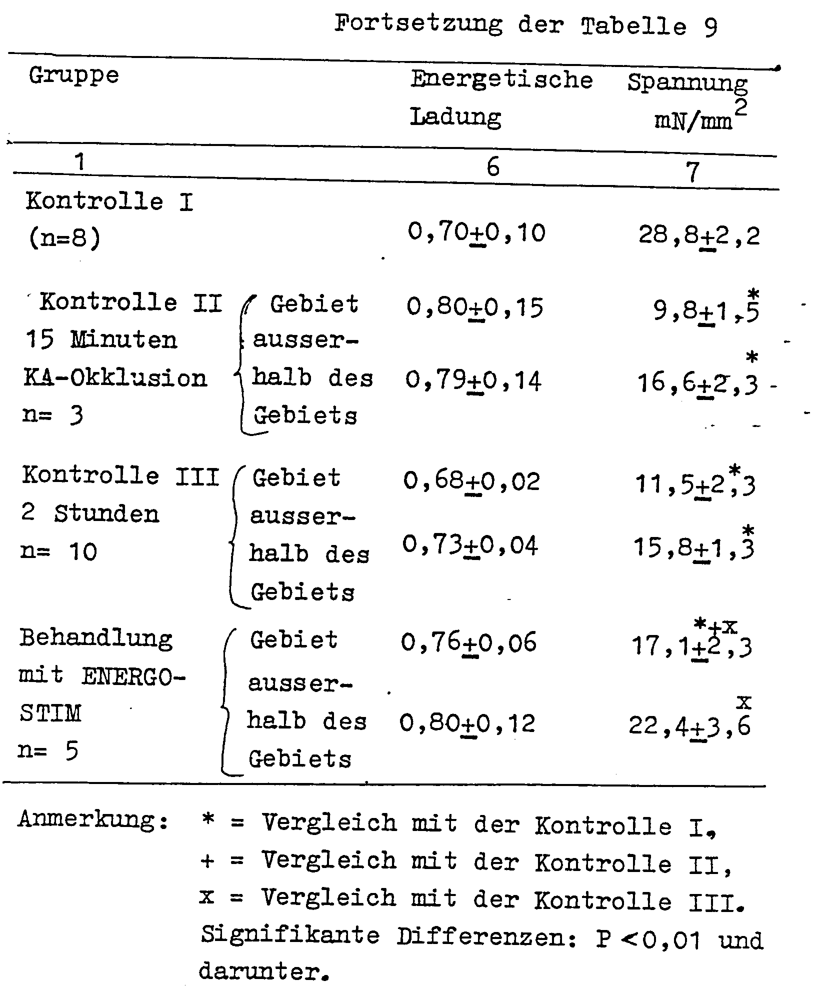

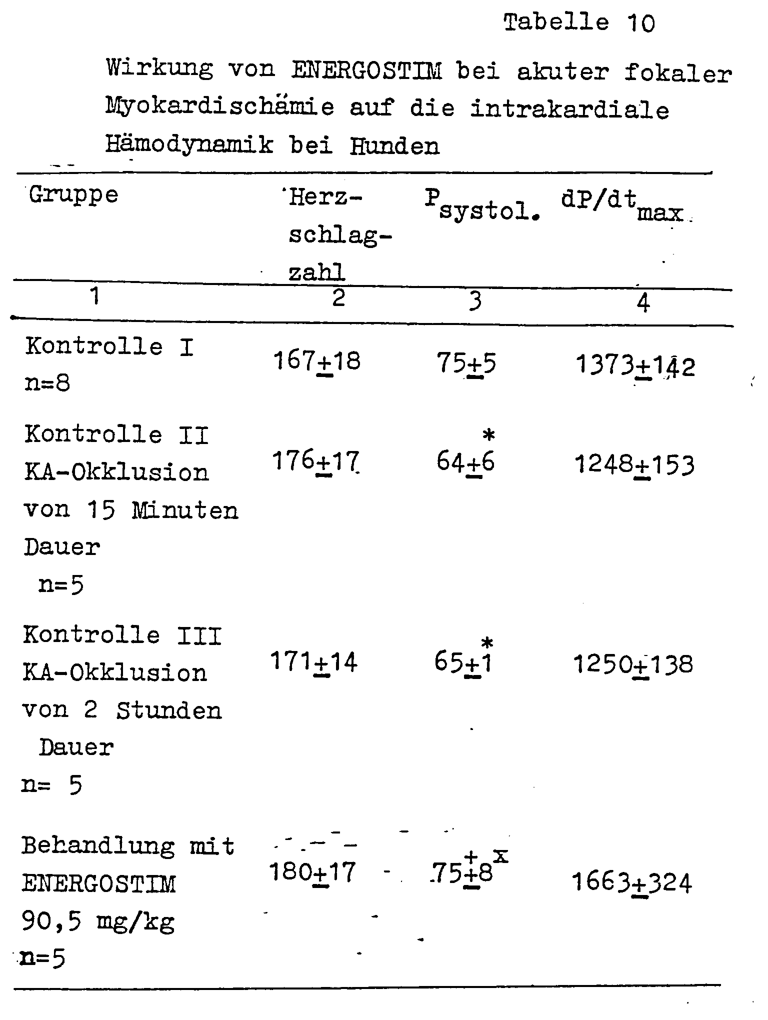

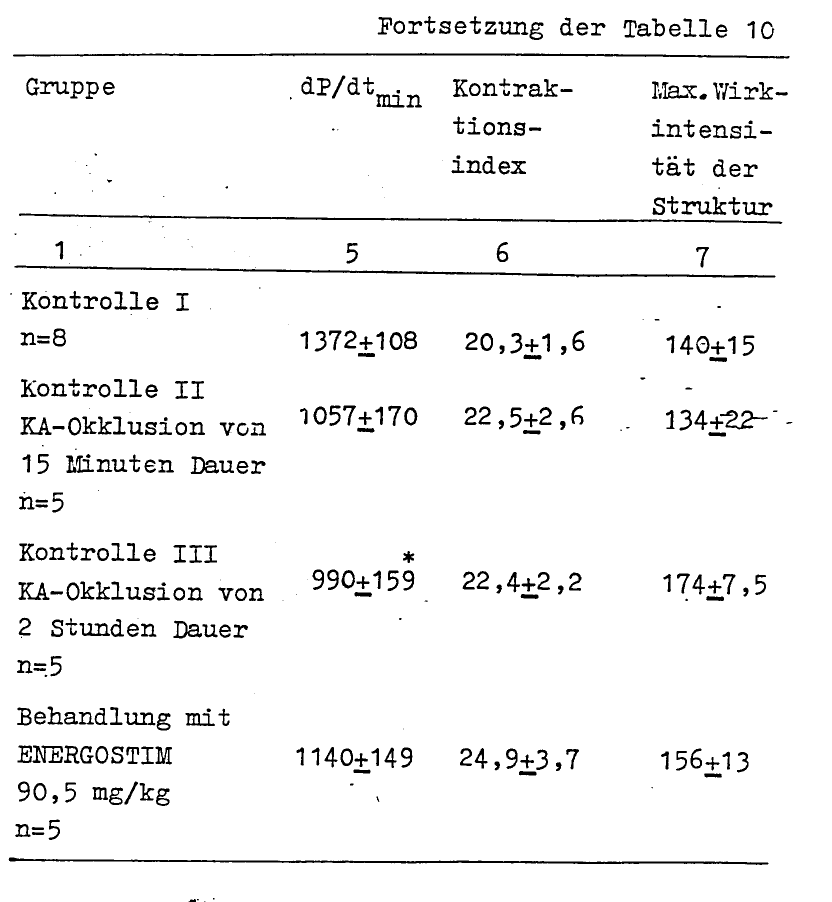

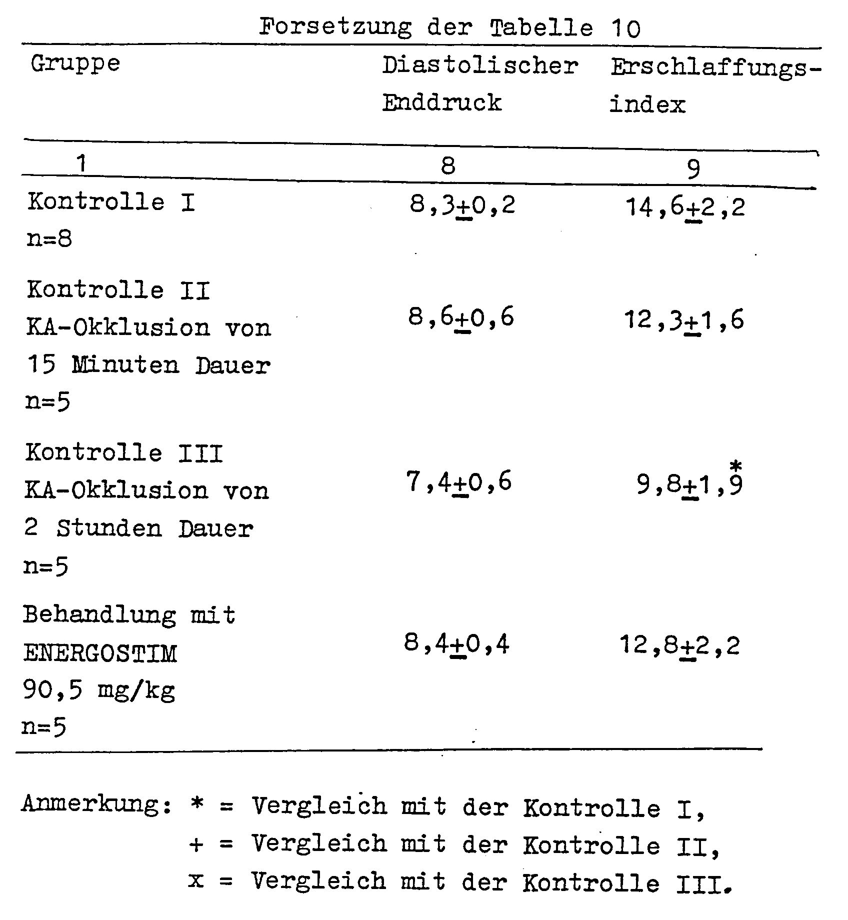

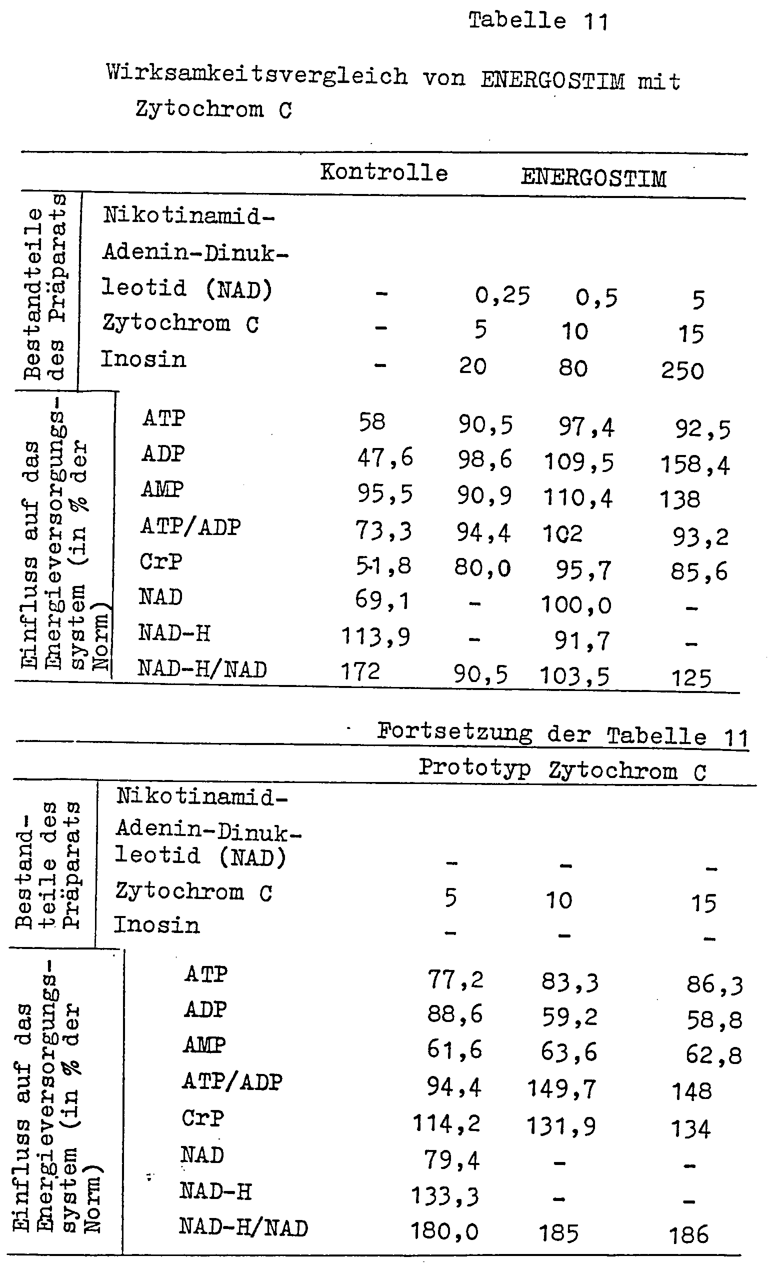

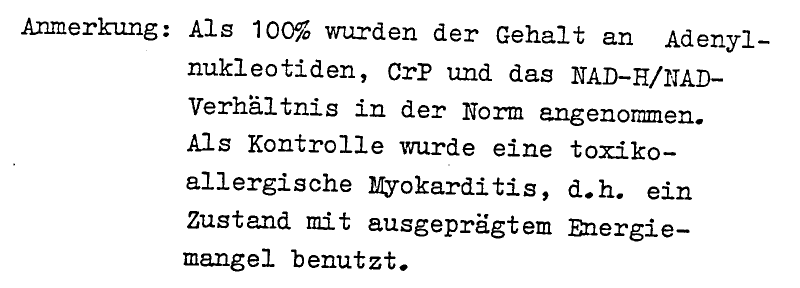

## Patentansprüche

1. Antihypoxisches Mittel auf der Basis von Zytochrom C,
**dadurch gekennzeichnet**, dass es zusätzlich Nikotinamid-Adenin-Dinukleotid und inosin bei folgendem Verhältnis der Bestandteile (Gew. Teile) enthält:
| | |
|---|---|
| Zytochrom C | 5 bis 15, |
| Nikotin-Adenin-Dinukleotid | 0.5 bis 5, |
| Inosin | 20 bis 250. |

## Claims

1. Antihypoxic agent based on cytochrome C, characterized in that it additionally contains nicotinamide adenine dinucleotide and inosine in the following ratio of components (parts by weight):
| | |
|---|---|
| cytochrome C | 5 to 15 |
| nicotine adenine dinucleotide | 0.5 to 5 |
| inosine | 20 to 250 |

## Revendications

1. Agent antihypoxique à base de cytochrome C caractérisé en ce qu'il contient en outre du nicotinamide-adénine-dinucléotide et de l'inosine dans les proportions suivantes des constituants (parties en masse) :
| | |
|---|---|
| cytochrome C | 5 à 15 |
| nicotine-adénine-dinucléotide | 0,5 à 5 |
| inosine | 20 à 250 |
